# EUROPEAN PATENT SPECIFICATION

(11) **EP 0 698 400 B1**
(45) Date of publication and mention of the grant of the patent: **29.03.2000**
(21) Application number: 95850137.1
(22) Date of filing: 08.08.1995
(51) Int. Cl.: A61N 1/05, A61B 5/042

(54) **Electrode device for intracardiac stimulation of heart tissue and/or sensing heart signals in a patient**
Elektrodenvorrichtung zur intrakardialen Stimulation des Herzgewebes und/oder zur Abnahme von Signalen des Herzens eines Patienten
Dispositif d'électrode pour stimulation du tissu intracardiaque et/ou détection des signaux cardiaques d'un patient

(30) Priority: 19.08.1994 SE 9402775
(43) Date of publication of application: 28.02.1996
(73) Proprietor: Pacesetter AB, 171 95 Solna (SE)
(72) Inventor: Lindegren, Ulf, S-121 33 Enskededalen (SE)
(74) Representative: Winblad, Hans Peter

(56) References cited:
- US-A- 4 289 138
- US-A- 4 567 901
- US-A- 4 848 352
- US-A- 5 306 292

## Description

The invention relates to an electrode device, for intracardiac stimulation of heart tissue and/or sensing heart signals in a patient, with an electrode cable containing at least two elongate, flexible conductors, insulated from each other, and with an electrode head, arranged at the distal end of the electrode cable and provided with at least two conductive surfaces, each connected to a separate conductor.

One such electrode device is known through the US patent document 5 306 292. In one embodiment, this electrode device's electrode head is equipped with a plurality of punctiform conductive surfaces. In a second embodiment, the electrode head consists of two conductive bodies, one of the bodies being a centrally arranged projecting part which is insulated from the other conductive body. In another embodiment in this US patent document, one of the conductive surfaces is arranged on the end of the electrode cable, and the second conductive surface is formed by part of a traumatic, helical fixation means on the electrode head.

US patent document 4 848 352 describes another pacemaker electrode, with a head subdivided into sections of conductive material insulated from each other, of the kind cited in the preamble.

The conductive surfaces in the cited electrode devices serve as stimulation surfaces and as sensing surfaces. In some of the embodiments presented here, the distance between the stimulation surfaces and, accordingly, even the distances between a stimulation surface and a sensing surface, can be varied when different conductive surfaces are connected by a pacemaker with a switching function. The distance between stimulation surfaces is governed to a large degree by the system's impedance, i.e. the greater the distance between stimulation surfaces, the greater the impedance obtained. This means that the physician might wish to reduce the distance between stimulation surfaces, if e.g. the threshold value is too high, so current increases and voltage decreases. In the prior art electrode devices cited here, the change in distance between stimulation surfaces on the electrode device's head is very limited, since the electrode cable can only hold a limited number of conductors, thereby limiting the number of conductive surfaces on the electrode head. Moreover, the change in distance is predetermined, since the conductive surfaces are permanently arranged on the electrode head with a fixed distance between them.

The purpose of the invention is to achieve an electrode device, in which the distance between the conductive surfaces can be varied continuously, simply and as needed, of the kind cited in the preamble.

This problem is solved by the features set forth in the characterizing clauses of the independent claims 1, 2 and 3.

With this structure, the physician can, after the electrode device has been implanted, use the control means to continuously vary the distance between the conductive surfaces and, using a measurement instrument, set the distance between the conductive surface with great accuracy to achieve an optimum threshold value for the patient.

Further features of the embodiments of claims 1-3 are defined in the dependent claims 4-6.

The invention will now be explained in greater detail, referring to the FIGURES in the attached drawings in which
FIGS. 1-4 show the distal ends of electrode devices in longitudinal section and in different embodiments according to the invention;
FIG. 5 shows a cross-section along the line V-V in FIG. 4 and
FIGS. 6 and 7 show the distal end of an electrode device in longitudinal section and in an additional embodiment according to the invention.

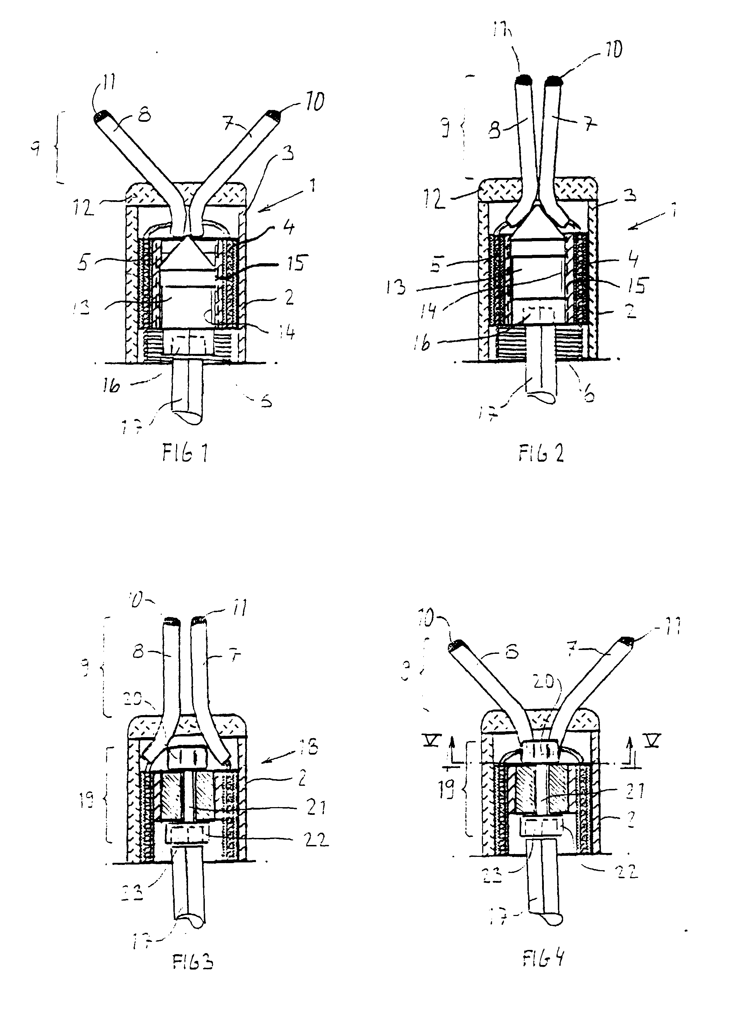

In FIG. 1 is shown the distal end of an electrode device 1 for intracardiac stimulation of heart tissue and/or sensing heart signals in a patient. The electrode device 1 consists of an electrode cable 2 with insulation 3 in which two elongate, flexible conductors 4, 5, insulated from each other, run. The conductors 4, 5 can preferably be helically wound parallel to each other and form the inner wall of a channel 6 extending the entire length of the electrode cable 2. Each conductor 4, 5 is connected to a pin-shaped part 7, 8 with a curved section and made of an electrically conductive material. These pin-shaped parts 7, 8 form an electrode head 9. The pins 7, 8 are for the most part covered by an insulated surface coating, except for their exposed distal ends which form conductive surfaces 10, 11 for stimulating heart tissue and/or sensing heart signals in a patient. An elastic, plastic material, e.g. silicone, in which the pins 7, 8 are resiliently mounted opposite each other, is provided at the distal end 12 of the electrode cable 2. In this FIGURE, the pins 7 and 8 are shown in a position in which they are not being acted on by any control means and in which the conductive surfaces 10, 11 are at a maximum distance from each other.

The electrode device is also equipped with a control means 13 which moves the pins 7, 8 and causes the distance between the conductive surfaces 10, 11 to change. The control means 13, which is arranged in the channel 6 immediately behind the proximal ends of the pins 7, 8, is cylindrical and provided with a thread 14 which matches a thread 15 arranged along a section of the channel. The distal end of the control means 13 is conical, and its proximal end is provided with a hexagonal socket 16 into which the distal end of a stylet 17 fits.

When a change in the distance between the conductive surfaces 10 and 11 is desired, the stylet 17 is inserted into the hexagonal socket 16. By rotating the stylet, the physician can then advance the control means 13, thereby pushing the conical end between the proximal ends of the pins 7, 8 and forcing the ends apart. The change in distance between the conductive surfaces 10 and 11 then depends on the distance the control means 13 is pushed between the ends of the pins 7, 8.

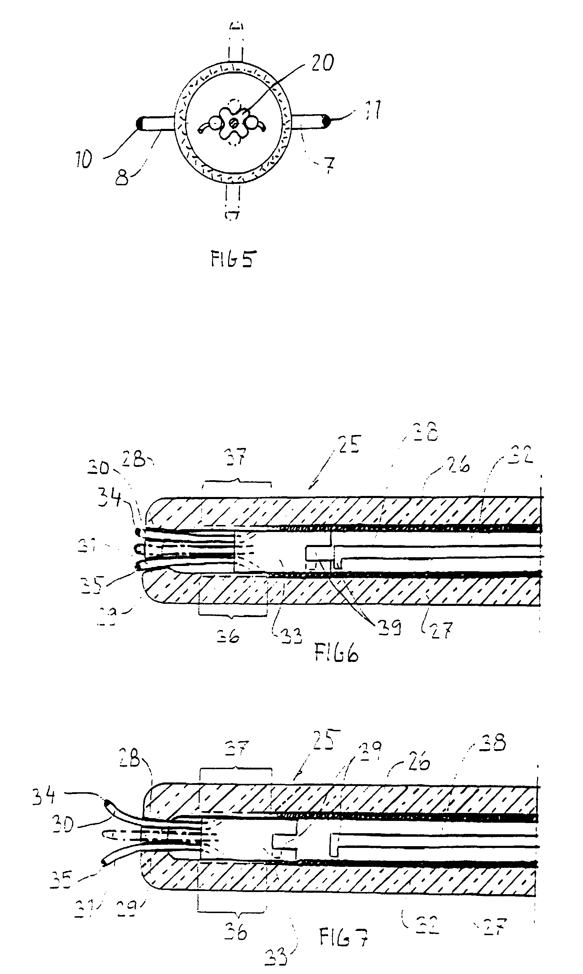

FIG. 2 shows the control means 13 maximally advanced between the ends of the pins 7, 8, so the distance between the conductive surfaces 10 and 11 is minimized in this embodiment. The maximum and minimum distances between the conductive surfaces 10, 11 are governed by the length and curvature of the pins 7, 8. FIG. 2 shows a position the pins 7, 8 can advantageously assume during the electrode device's implantation, since the pins in this position do not laterally protrude outside the electrode cable 2.

If the positions shown for the pins 7, 8 in FIG. 1 create an optimum distance between the conductive surfaces 10, 11 for stimulating heart tissue, the pins 7, 8 in this position can serve as fixation means for the electrode cable 2. This embodiment only shows and describes two pins 7, 8, each with one conductive surface 10, 11. The electrode head 9 could naturally consist of a large number of such pins 7, 8, each with a conductive surface, said pins being resiliently mounted at their ends 12 and adjustable with the common control means 13.

FIGS. 3 and 4 show the distal end of an electrode device 18 which only differs from the electrode device 1 in FIGS. 1 and 2 by having a different control means. So the electrode device's 18 parts, except for the control means, have the same reference designations as in FIGS. 1 and 2. The control means, assigned the reference designation 19 when associated with the electrode device 18, consists of an eccentric cam 20 which is rotatingly arranged in the channel 6 between the rear ends of the pins 7, 8. The eccentric cam 20 is connected by a shaft 21 to a rotation means 22 with a hexagonal socket 23 into which the stylet 17 can be inserted. The eccentric cam 20 and the rotation means 22 with the shaft 21 are rotatingly arranged in a holder 24 attached to the surrounding channel wall. When the physician wishes to change the distance between the conductive surfaces 10 and 11, she/he inserts the stylet into the hexagonal socket 23 of the rotation means 22 and rotates the stylet 17 in some direction. The peripheral surface of the eccentric cam 20 then displaces the rear ends of the pins 7, 8, thereby changing the distance between the distal ends bearing the conductive surfaces 10, 11. In FIG. 3, the eccentric cam 20 has been turned to a position in which the rear ends of the pins 7, 8 touch the part of the peripheral surface at the greatest distance from the eccentric cam's shaft 21. In this position, the conductive surfaces 10, 11 are separated by a minimum distance. The electrode cable 2 can advantageously be implanted with the pins in this position.

FIG. 4 shows a pin position in which the conductive surfaces 10, 11 are arranged at a maximum distance from each other. The rear ends of the pins 7, 8 now touch the part of the eccentric cam's 20 peripheral surface closest to the cam's shaft 21. In this pin position, the pins 7, 8 can even serve as fixation means.

FIG. 5 is a section along the line V-V in FIG. 4. This view shows the shape of the eccentric cam 20. The FIG. also shows that the eccentric cam 20 can control more pins than the pins 7, 8 described. Two additional pins are indicated here with dotted lines. The shape of the eccentric cam 20 is not restricted to the one shown but can differ, depending on the number of pins and the way the pins are to be displaced in relation to each other.

FIGS. 6 and 7 present an electrode device 25 which differs from the electrode devices 1 and 18 illustrated and described above. This electrode device 25 has an electrode cable 26 with a sleeve of insulation 27 which also seals off the distal end of the electrode cable 26, except for a number of channel-like holes 28, 29. This electrode device 25 also has a channel 32 which runs the entire length of the electrode cable 26. A cylindrical control means 33 is slidingly arranged in the channel 32 immediately behind the end of the electrode cable 26. Pin-shaped parts 30, 31 with conductive surfaces 34, 35 are arranged at the distal end of the control means 33. Each of the pins 30, 31 runs in a separate channel 28, 29, the channels 28, 29 being arrayed in such a way that longitudinal movement of the control means 33 and the pins 30, 31 respectively changes the distance between the conductive surfaces 34 and 35. The pins 30, 31 can even be precurved so a relatively long distance is achieved between the conductive surfaces 34, 35 when pin protrusion out of the channels 28, 29 is at a maximum. Each of the pins 30, 31 can preferably be connected across the cylindrical control means 33 to a separate conductor forming the inner wall of the channel 32 and whose ends are exposed as contact rails along a stretch 36, 37, the proximal ends of the pins 30, 31 then being in constant contact with these contact rails. The control means is moved with a stylet 38. The stylet 38 and the control means 33 are interconnected with e.g. a bayonet lock 39. The external diameter of the control means 33 is such, in relation to the diameter of the channel 32, that the control means 33 can only be moved with the stylet 38. In FIG. 6 the pins 30, 31 have been moved so the distance between conductive surfaces 34, 35 is at a minimum. The pins 30, 31 can also be fully retracted into the channels 28, 29, thereby facilitating implantation of the electrode device. In FIG. 7, the pins 30, 31 have been advanced, thereby changing the distance between the conductive surfaces 34, 35. If the control means 33 is advanced even further, a longer distance is achieved between the said conductive surfaces 34, 35. This electrode device 25 can also be equipped with a large number of pins with attendant conductive surfaces, as designated by the dash-dotted outline of a pin. Even in this embodiment, the electrode head consists of the pins 30, 31.

Other embodiments of the electrode device are conceivable within the scope of the invention. The main feature is that the distance between the described conductive surface is continuously variable, making it possible to achieve an optimum threshold value for the patient. The electrode device according to the invention can preferably be connected to a pacemaker with a switching function with which the different conductive surfaces can be connected as desired so the distance can be varied between the stimulation surfaces, and accordingly, the distance between a stimulation surface and a sensing surface.

## Claims

1. An electrode device, for intracardiac stimulation of heart tissue and/or sensing heart signals in a patient, with an electrode cable (2) containing at least two elongate, flexible conductors (4,5), insulated from each other, and with an electrode head (9), arranged at the distal end of the electrode cable (2) and provided with at least two conductive surfaces (10,11), each connected to a separate conductor, **characterized in** that the electrode head (9) consists of at least two parts (7,8) movable in relation to each other, each part (7,8) being provided with at least one conductive surface (10,11), and the electrode device (1) is equipped with a control means (13), which is axially movable in the distal end of the electrode cable (2) and having a conical distal end for engagement with and lateral displacement of proximal end portions of said parts (7,8) so as to vary the distance between the conductive surfaces (10,11).

2. An electrode device, for intracardiac stimulation of heart tissue and/or sensing heart signals in a patient, with an electrode cable (2) containing at least two elongate, flexible conductors, insulated from each other, and with an electrode head (9), arranged at the distal end of the electrode cable (2) and provided with at least two conductive surfaces (10,11), each connected to a separate conductor, **characterized in** that the electrode head (9) consists of at least two parts (7,8) movable in relation to each other, each part (7,8) being provided with at least one conductive surface (10,11), and the electrode device (18) is equipped with a control means (19), which consists of an eccentric cam (20) rotatably arranged between proximal end portions of said parts (7,8) for lateral displacement thereof and variation of the distance between the conductive surfaces (10,11).

3. An electrode device, for intracardiac stimulation of heart tissue and/or sensing heart signals in a patient, with an electrode cable (26) containing at least two elongate, flexible conductors, insulated from each other, and with an electrode head (30,31), arranged at the distal end of the electrode cable (26) and provided with at least two conductive surfaces (34,35), each connected to a separate conductor, **characterized in** that the electrode head consists of at least two parts (30,31) movable in relation to each other, each part (30,31) being provided with at least one conductive surface (34,35), and the electrode device (25) is equipped with a control means (33), which is axially slidable at the distal end of the electrode cable (26) and connected to proximal end portions of said parts (30,31) for displacement of said parts through channels (28,29) at the distal end of the electrode cable (26) thereby enabling variations of the distance between the conductive surfaces (34,35).

4. An electrode device according to any of claims 1-3, **characterized in** that each part (7,8,30,31) of the electrode head (9,30,31) consists of a pin, made of an electrically conductive material, which constitutes the conductive surface (10,11,34,35) at least in part.

5. An electrode device according to claim 1, 2 or 4, **characterized in** that the movable parts (7,8) are resiliently mounted opposite each other, at an elastic end portion (12) of the electrode cable (2).

6. An electrode device according to any of claim 1, 2, 4 or 5, **characterized in** that at least one of the parts (7,8) is provided with a curved section.

## Patentansprüche

1. Eine Elektrodenvorrichtung zur intrakardialen Stimulation von Herzgewebe und/oder zum Abfühlen von Herzsignalen in einem Patienten mit einem Elektrodenkabel (2), das zumindest zwei langgestreckte, flexible Leiter (4,5) enthält, die voneinander isoliert sind, und mit einem Elektrodenkopf (9), der am distalen Ende des Elektrodenkabels (2) angeordnet ist und mit mindestens zwei leitenden Oberflächen (10,11) versehen ist, von denen jede mit einem separaten Leiter verbunden ist, **dadurch gekennzeichnet**, daß der Elektrodenkopf (9) aus zumindest zwei Teilen (7,8) besteht, die in Beziehung zueinander bewegbar sind, wobei jeder Teil (7,8) zumindest mit einer leitenden Oberfläche (10,11) versehen ist, und daß die Elektrodenvorrichtung (1) mit einem Steuermittel (13) ausgestattet ist, das am distalen Ende des Elektrodenkabels (2) axial bewegbar ist und ein konisches distales Ende zum Zusammenwirken und lateralen Verschieben von proximalen Endteilen dieser Teile (7,8) hat, um den Abstand zwischen den leitenden Oberflächen (10,11) zu variieren.

2. Eine Elektrodenvorrichtung zur intrakardialen Stimulation von Herzgewebe und/oder Abfühlen von Herzsignalen in einem Patienten mit einem Elektrodenkabel (2), das zumindest zwei langgestreckte, flexible Leiter (4,5) enthält, die voneinander isoliert sind, und mit einem Elektrodenkopf (9), der am distalen Ende des Elektrodenkabels (2) angeordnet ist und mit mindestens zwei leitenden Oberflächen (10,11) versehen ist, von denen jede mit einem separaten Leiter verbunden ist, **dadurch gekennzeichnet**, daß der Elektrodenkopf (9) aus zumindest zwei Teilen (7,8) besteht, die in Beziehung zueinander bewegbar sind, wobei jeder Teil (7,8) mit zumindest einer leitenden Oberfläche (10,11) versehen ist, und daß die Elektrodenvorrichtung (18) mit einem Steuermittel (19) ausgestattet ist, das aus einem exzentrischen Nocken (20) besteht, der drehbar zwischen proximalen Endabschnitten dieser Teile (7,8) zur lateralen Verschiebung derselben und Variationen des Abstandes zwischen den leitenden Oberflächen (10,11) angeordnet ist.

3. Eine Elektrodenvorrichtung zur intrakardialen Stimulation von Herzgewebe und/oder zum Abfühlen von Herzsignalen in einem Patienten mit einem Elektrodenkabel (26), das zumindest zwei langgestreckte, flexible Leiter enthält, die voneinander isoliert sind, und mit einem Elektrodenkopf (30,31), der am distalen Ende des Elektrodenkabels (26) angeordnet ist und mit mindestens zwei leitenden Oberflächen (34,35) versehen ist, von denen jede mit einem separaten Leiter verbunden ist, **dadurch gekennzeichnet,** daß der Elektrodenkopf aus zumindest zwei Teilen (30,31) besteht, die in Beziehung zueinander bewegbar sind, wobei jeder Teil (30,31) zumindest mit einer leitenden Oberfläche (34,35) versehen ist, und daß die Elektrodenvorrichtung (25) mit einem Steuermittel (33) ausgestattet ist, das am distalen Ende des Elektrodenkabels (26) axial gleitbar ist und mit proximalen Endabschnitten dieser Teile (30,31) zur Verschiebung dieser Teile durch Kanäle (28,29) an dem distalen Ende des Elektrodenkabels (26) verbunden ist, wodurch Variationen des Abstandes zwischen den leitenden Oberflächen (34,35) ermöglicht werden.

4. Eine Elektrodenvorrichtung nach einem der Ansprüche 1 - 3, **dadurch gekennzeichnet,** daß jeder Teil (7,8,30,31) des Elektrodenkopfes (9,30,31) aus einem Stift besteht, der aus elektrisch leitendem Material, das zumindest teilweise die leitende Oberfläche (10,11,34,35) bildet, hergestellt ist.

5. Eine Elektrodenvorrichtung nach Anspruch 1,2 oder 4, **dadurch gekennzeichnet,** daß die bewegbaren Teile (7,8) federnd sich gegenüberstehend an einem elastischen Endteil (12) des Elektrodenkabels (2) angebracht sind.

6. Eine Elektrodenvorrichtung nach einem der Ansprüche 1,2,4 oder 5, **dadurch gekennzeichnet,** daß zumindest einer der Teile (7,8) mit einer gekrümmten Sektion versehen ist.

## Revendications

1. Dispositif d'électrode, pour une stimulation intracardiaque de tissus cardiaques et/ou une détection de signaux cardiaques chez un patient, comportant un câble (2) d'électrode contenant au mois deux conducteurs (4, 5) oblongs souples, isolés l'un de l'autre, et comportant une tête (9) d'électrode, disposée à l'extrémité distale du câble (2) d'électrode et munie d'au moins deux surfaces (10, 11) conductrices, chacune reliée à un conducteur distinct, caractérisé en ce que la tête (9) d'électrode est constituée d'au moins deux parties (7, 8) mobiles l'une par rapport à l'autre, chaque partie (7, 8) étant munie d'au moins une surface (10, 11) conductrice, et le dispositif (1) d'électrode est muni de moyens (13) de commande, qui sont mobiles axialement dans l'extrémité distale du câble (2) d'électrode et qui ont une extrémité distale conique pour obtenir une coopération avec des parties d'extrémité proximales des parties (7, 8) et un déplacement latéral de ces parties d'extrémité proximales des parties (7, 8) de manière à modifier la distance entre les surfaces (10, 11) conductrices.

2. Dispositif d'électrode pour une stimulation intracardiaque de tissus cardiaques et/ou une détection de signaux cardiaques chez un patient, comportant un câble (2) d'électrode contenant au moins deux conducteurs oblongs souples, isolés l'un de l'autre, et comportant une tête (9) d'électrode, disposée à l'extrémité distale du câble (2) d'électrode et munie d'au moins deux surfaces (10, 11) conductrices, chacune reliée à un conducteur distinct, caractérisé en ce que la tête (9) d'électrode est constituée d'au moins deux parties (7, 8) mobiles l'une par rapport à l'autre, ces parties (7, 8) étant munies d'au moins une surface (10, 11) conductrice, et le dispositif (18) d'électrode est muni de moyens (19) commande, qui sont constitués d'une came (20) à excentrique montée de manière rotative entre des parties d'extrémité proximales des parties (7, 8) pour obtenir leur déplacement latéral et une variation de la distance entre les surfaces (10, 11) conductrices.

3. Dispositif d'électrode, pour une stimulation intracardiaque des tissus cardiaques et/ou une détection des signaux cardiaques chez un patient, comportant un câble (26) d'électrode contenant au moins deux conducteurs oblongs souples, isolés l'un de l'autre, et comportant une tête (30, 31) d'électrode disposée à l'extrémité distale du câble (26) d'électrode et munie d'au moins deux surfaces (34, 35) conductrices, chacune reliée à un conducteur distinct, caractérisé en ce que la tête d'électrode est constituée d'au moins deux parties (30, 31) mobiles l'une par rapport à l'autre, chaque partie (30, 31) étant munie d'au moins une surface (34, 35) conductrice, et le dispositif (25) d'électrode est muni de moyens (33) de commande, qui sont coulissants de manière axiale à l'extrémité distale du câble (26) d'électrode et reliés à des parties d'extrémité proximales des parties (30, 31) pour obtenir un déplacement de ces parties dans les canaux (28, 29) à l'extrémité distale du câble (26) d'électrode, pour permettre ainsi des modifications de la distance entre les surfaces (34, 35) conductrices.

4. Dispositif d'électrode suivant l'une quelconque des revendications 1 à 3, caractérisé en ce que chaque partie (7, 8, 30, 31) de la tête (9, 30, 31) d'électrode est constituée d'une broche, en un matériau conducteur d'électricité, qui constitue la surface (10, 11, 34, 35) conductrice au moins en partie.

5. Dispositif d'électrode suivant la revendication 1, 2 ou 4, caractérisé en ce que les parties (7, 8) mobiles sont montées élastiquement opposées l'une à l'autre, à une partie (12) d'extrémité élastique du câble (2) d'électrode.

6. Dispositif d'électrode suivant l'une quelconque des revendications 1, 2, 4 ou 5, caractérisé en ce qu'au moins une des parties (7, 8) est munie d'une section incurvée.
